# EUROPEAN PATENT APPLICATION

(11) **EP 4 737 549 A1**
(43) Date of publication of application: **06.05.2026**
(21) Application number: 24210255.6
(22) Date of filing: 31.10.2024
(51) Int. Cl.: C12M 1/00, A61M 1/36, C12M 1/42, C12M 1/34, C12M 1/36, G01N 33/49, A61M 1/34, G01N 15/1433, G01N 15/14, G01N 15/10

(54) **CELL ENGINEERING MODULE**

(71) Applicant: Imec VZW, 3001 Leuven (BE)
(72) Inventor: STAKENBORG, Tim, 3001 Heverlee (BE); STIRPARO, Rocco, 3000 Leuven (BE); PEUMANS, Peter, 1540 Herfelingen (BE)
(74) Representative: Winger

(57) **Abstract**

In a one aspect, the present invention relates to a cell engineering module (4) for cell engineering target cells (51) in a mixture (5) comprising the target cells (51) and non-target cells (52), comprising: i) a cell monitoring zone (410) for allowing tracking and classifying individual cells (50) in the mixture (5), wherein classifying a cell (50) comprises determining whether the cell (50) is a target cell (51); and ii) an engineering component (42) in the cell monitoring zone (410) for selectively modifying individual cells (50) at one or more engineering sites (420), wherein the target cells (51) and the non-target cells (52) are randomly distributed in the engineering component (42); wherein the engineering component (42) is adapted for-in operation-being controlled so as to selectively modify only target cells tracked to be at one of the engineering sites (420).

## Description

### Technical field of the invention

The present invention relates to the field of cell engineering, and more specifically to selectively modifying individual target cells within a mixture of target and non-target cells.

### Background of the invention

In the field of cellular biology and medical research, the ability to modify (e.g. genetically) cells at the single-cell level is crucial e.g. for advancements in therapy and for furthering our understanding of cellular functions. Traditional approaches to modifying target cells in a mixture of cells typically involve sorting out the target cells, followed by transduction or transfection of the target cells. These approaches thus typically require the physical separation of target cells from a heterogeneous mixture, which can be inefficient and yield less than optimal results. Moreover, these approaches generally handle cells in batches rather than on an individual basis, limiting control in cell modification tasks.

Additionally, sorting is generally done using label-based methods, such fluorescence-activated cell sorting (FACS) or magnetic-activated cell sorting (MACS). These techniques, while effective for certain applications, require the cells to be labelled (e.g. with fluorescent markers for FACS or magnetic beads for MACS). This labelling process not only adds additional steps (e.g. additional sample preparation and wash steps to remove unbound labels or reagents) to the procedure-making it time-consuming, complex and more at risk of cell loss or damage-but also introduces foreign materials that can alter cell behaviour/function and/or cell viability. These issues become exceedingly critical when dealing with rare cell types or when sample sizes are limited.

One area in which cell engineering is particularly useful is that of cell therapy. Our immune system plays a pivotal role in defending the body against infections and diseases. It possesses the remarkable ability to adapt and respond to various pathogens and internal threats, including viruses, bacteria, cancerous cells, fibrosis and aging. However, there are instances where the immune system fails to make the right tool to fight back (e.g. for HIV), is too slow and needs to be prepared upfront (e.g. with vaccines), or struggles when the threat is an alteration of our own cells (e.g. in the case of cancer or aging).

To bolster the immune system's capacity to fight diseases, various strategies have been developed to prime the immune system for fighting a disease of interest. For example, vaccination is a well-known method that prepares the immune system in advance to combat infections, such as influenza or COVID-19. Another advanced approach involves cell therapies, such as chimeric antigen receptor T (CAR-T) cell therapies, which entail modifying the receptors of T-cells to specifically bind to a particular target antigen on certain cells (e.g. cancer cells). While CAR-T therapies have demonstrated promising results, especially in treating previously untreatable cancers, the production of these therapies is fraught with challenges.

The current manufacturing processes for cell therapies are complex and time-consuming, often requiring several weeks to produce a single dose. This involves multiple steps such as cell extraction, selection, modification, and expansion, each necessitating specialized equipment, facilities and personnel. Furthermore, extensive quality control tests at each stage contribute to the overall cost and duration of therapy production. The logistics involved further complicate accessibility, as most manufacturing facilities are centralized, necessitating the transportation of biological materials over long distances, potentially compromising their viability and efficacy.

Additionally, cell engineering is also extensively used in lab/research settings; e.g. in gene expression studies, knock-down/out studies, protein production, stem cell research, high-throughput screening, miRNA studies, etc

There is thus still a need in the art for ways to modify target cells which address at least some of the issues outlined above.

### Summary of the invention

It is an object of embodiments of the present invention to provide an apparatus for selectively modifying individual target cells in a mixture comprising the target cells and non-target cells. This objective is accomplished by systems, modules, cartridges and methods according to the present invention.

The claimed invention is as set out in the appended claims.

It is an advantage of embodiments of the present invention that they are capable of selectively modifying target cells, ensuring that only the targeted cells are modified. It is a further advantage of embodiments of the present invention that they allow for the modification of target cells without the necessity of isolating them from a mixture comprising both target and non-target cells. It is yet a further advantage of embodiments of the present invention that they can enable the classification and selection of cells based without the need for external labels such as magnetic beads or fluorescent tags, which simplifies the process and reduces the risk of contamination.

It is an advantage of embodiments of the present invention that the cell monitoring and engineering can occur in a continuous flow, and even at a relatively high cell-throughput. Such continuous flow can be a constant flow or a (non-constant) modulated flow, e.g. by surface modification as described in EP24205936.8, which is incorporated herein by reference. It is a further advantage of embodiments of the present invention that decisions regarding the cells to be modified are made at the single-cell level, allowing for precise and targeted cell engineering.

It is an advantage of embodiments of the present invention that multiple individual cells can be classified and tracked in parallel, significantly increasing efficiency. It is a further advantage of embodiments of the present invention is that cell monitoring can be performed in real-time over a relatively wide area.

It is an advantage of embodiments of the present invention that the entire process can be completed in a closed loop system, maintaining the integrity and sterility of samples throughout the process. This not only enhances safety but also reduces the quality control that must be performed.

It is an advantage of embodiments of the present invention that they can be beneficially used for both therapeutic (e.g. cell therapy) and non-therapeutic (e.g. research) applications.

It is an advantage of embodiments of the present invention that they can be performed in a decentralized manner (e.g. directly at the point of care or in a laboratory), which eliminates the need for transportation of samples and/or long travel times for the patient and/or researcher.

Particular and preferred aspects of the invention are set out in the accompanying independent and dependent claims. Features from the dependent claims may be combined with features of the independent claims and with features of other dependent claims as appropriate and not merely as explicitly set out in the claims.

Although there has been constant improvement, change and evolution of devices in this field, the present concepts are believed to represent substantial new and novel improvements, including departures from prior practices, resulting in the provision of more efficient, stable and reliable devices of this nature.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

FIG 1 schematically depicts a system and a cell engineering module, in accordance with the present invention.
FIG 2 schematically depicts a system which incorporates a cell engineering module, in accordance with illustrative embodiments of the present invention.
FIG 3 schematically depicts a cell engineering module in the form of a cartridge and a device for receiving said cartridge, in accordance with illustrative embodiments of the present invention.
FIG 4 schematically depicts a more detailed top view of a potential cell engineering , imaging unit and control component, in accordance with illustrative embodiments of the present invention.
FIG 5 schematically depicts a side view of the cell engineering module and control component of FIG 4.
FIG 6 schematically depicts a more detailed top view of a further potential cell engineering module, imaging unit and control component, in accordance with illustrative embodiments of the present invention.
FIG 7 schematically depicts a side view of the cell engineering module and control component of FIG 6.
FIG 8 is a block diagram of a vein-to-vein system, in accordance with illustrative embodiments of the present invention.
FIG 9 is a block diagram of a lab system, in accordance with illustrative embodiments of the present invention.

In the different figures, the same reference signs refer to the same or analogous elements.

### Description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second, third and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable with their antonyms under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. The term "comprising" therefore covers the situation where only the stated features are present and the situation where these features and one or more other features are present. Thus, the scope of the expression "a device comprising means A and B" should not be interpreted as being limited to devices consisting only of components A and B. It means that with respect to the present invention, the only relevant components of the device are A and B.

Similarly, it is to be noticed that the term "coupled", also used in the claims, should not be interpreted as being restricted to direct connections only. The terms "coupled" and "connected", along with their derivatives, may be used. It should be understood that these terms are not intended as synonyms for each other. Thus, the scope of the expression "a device A coupled to a device B" should not be limited to devices or systems wherein an output of device A is directly connected to an input of device B. It means that there exists a path between an output of A and an input of B which may be a path including other devices or means. "Coupled" may mean that two or more elements are either in direct physical or electrical contact, or that two or more elements are not in direct contact with each other but yet still co-operate or interact with each other.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practised without these specific details. In other instances, well-known methods, structures and techniques have not been shown in detail in order not to obscure an understanding of this description.

The following terms are provided solely to aid in the understanding of the invention.

As used herein, and unless otherwise specified, the term 'cell engineering' refers to the purposeful process of modifying a (living) cell. This may generally include inserting, deleting and/or altering a component in the cell. In specific instances, said component may be a genetic sequence, such as a nucleic acid sequence (e.g. deoxyribonucleic acid, DNA; or ribonucleic acid, RNA). This may for example be achieved by genetic transformation. Within the present invention, the cell which is modified may be a prokaryote or an eukaryote, though more typically a eukaryote.

As used herein, and unless otherwise specified, the term 'genetic transformation' refers to the genetic modification/alteration of a cell resulting from the direct uptake and incorporation of genetic material-typically exogenous/foreign genetic material-from its surroundings through the cell membrane(s). Specific example of genetic transformation include transduction-by which the genetic material is introduced into the cell by a virus or viral vector-or transfection-by which the genetic material is introduced into the cell by non-viral means. Transfection can for example be achieved through opening of the cell (herein also referred to as 'poration') by physical means (e.g. electroporation, photoporation, sonoporation, cell squeezing or magnetofection) or (bio)chemical means (e.g. calcium phosphate, lipofection, etc.).

As used herein, and unless otherwise specified, the term 'target cell' refers to a cell of interest which is to undergo cell engineering. By contrast, a 'non-target cell' refers to a cell which isn't a target for the engineering (and should not undergo the modification). Within the present invention, cell engineering is generally performed in a mixture comprising both target cells and non-target cells.

As used herein, and unless otherwise specified, the term 'cell monitoring' refers to a of tracking and classifying individual cells in a mixture. Here, 'classifying a cell' comprises determining whether the cell is a target cell. Cell monitoring may typically be performed within a cell monitoring zone, using an imaging unit. A 'cell monitoring component' is component capable of performing cell monitoring. Such a cell monitoring component may thus typically comprise a cell monitoring zone and an imaging unit. A specific type of cell monitoring is 'label-free cell monitoring', wherein the cell monitoring takes place without the use of labels/tags/markers (such as fluorescent dyes, antibodies, magnetic beads, or any other exogenous substances by which the cells may be labelled).

As used herein, and unless otherwise specified, the term 'computational imaging' refers to using computation as part of the image formation process or to reveal data-driven insights using algorithms from captured data. In other words, computational imaging relies on algorithms and models to construct, modify or interpret a captured image (i.e. using optics) or even form images from measurements. Such processes may often rely on a significant amount of computing, but often allow to reconstruct/deduce information that traditional cameras cannot capture and/or is not present in an original image. An example is holographic imaging which uses interference of light waves to capture a hologram and subsequently computationally process said hologram. In general, computational imaging can involve reconstructing an actual image, or processing raw data to deduce imaging information therefrom without actually reconstructing an image as such. For example in holographic imaging, the measurement may be the capture of an interference pattern (i.e. a hologram; e.g. using a digital image sensor), from which a 3D detailed image (revealing the shape, size, location, etc. of the objects yielding said interference pattern) is computationally reconstructed. Alternatively, no image as may be reconstructed , but instead the necessary information (e.g. size, location, etc.) on the objects (e.g. cells) is derived directly from the measured hologram. Examples of other (label-free) computational imaging include high-resolution ptychographic imaging, 3D ptychographic imaging, optical diffraction tomography microscopy and label-free structured illumination microscopy. Compared to traditional imaging (i.e. using optics to capture an image as such, without computational enhancement), computational imaging creates or enhances the imaging to a level that one cannot achieve relying on optics alone.

As used herein, and unless otherwise specified, the term 'engineering component' refers to a component capable of performing cell engineering on a cell. Within the present invention, said engineering component is more specifically capable of selectively (e.g. under control by the control component) modifying-e.g. by genetic transformation (cf. supra)-individual cells at one or more engineering sites.

As used herein, and unless otherwise specified, the term 'control component' refers to a component capable of controlling another component or system. For example, a control component may be capable of controlling the engineering component, such as controlling an activation thereof. The latter may-as the case may be-for instance include one or more of controlling the triggering of an electric, light, sound or magnetic pulse (e.g. to open the cell), or controlling the release of one or more (bio)chemicals (e.g. a (bio)chemical for opening the cell and/or a nucleic acid to be inserted into the cell). To this end, the control component may typically comprise a processor or other similar processing means configured for performing its control function. Moreover, the control component may typically enact its control function based on one or more inputs, and thus the control component may comprise a processor or other similar processing means (the same or a different one) to process these inputs.

As used herein, and unless otherwise specified, the term 'cell movement modifying surface' refers to a surface capable of interacting distinctly with different types of cells, such that a trajectory of some cell types is distinctly altered as compared to other cell types. The cell movement modifying surface may for example have a higher affinity (e.g. a physical, chemical and/or biochemical affinity) for one type of cell as compared to another type of cell.

As used herein, and unless otherwise specified, the term 'cell type' (or 'cell category') refers to any cell categorization as may be useful in the context. For instance, one cell type could be all cells which have a certain expression level of a marker, or of a combination of multiple markers. Another cell type could be all cells having a certain physical morphology (e.g. shape and/or size).

As used herein, and unless otherwise specified, the term 'biological mixture' refers to a mixture comprising biological entities (e.g. cells, organelles, nucleic acids, proteins, carbohydrates, lipids, etc.). Such a mixture may be of biological origin (e.g. a bodily fluid) or artificial origin (e.g. cells in a buffer).

As used herein, and unless otherwise specified, the term 'apheresis' refers to a process by which a particular substance or component is removed from blood. Herein, the substance or component is further referred to as the 'fraction of interest' and typically comprises target cells; while the rest-i.e. the blood from which the fraction of interest has been separated-is referred to as the 'residual fraction'. The residual fraction may be returned to the body/patient. In some instances, also the fraction of interest is-after cell engineering-returned to the body/patient. The latter may be done together with or separate from the residual fraction. Specific types of apheresis include plasmapheresis (removing blood plasma), erythrocytapheresis (removing red blood cells), plateletpheresis (also referred to as 'thrombapheresis' or 'thrombocytapheresis', removing blood platelets), leukapheresis (removing leukocytes/white blood cells), lymphapheresis (removing lymphocytes) and stem cell harvesting (removing hematopoietic stem cells).

As used herein, and unless otherwise specified, the term 'vein-to-vein system' refers to a system the operation of which starts with drawing blood from a patient ('extraction'; e.g. typically from a vein) and ends with transferring modified target cells back into a patient ('reinsertion'; the patent may be the same or a different patient). In other words, the blood is temporarily withdrawn, not permanently removed. In preferred embodiments, this may be performed in a 'closed loop', wherein the extracted blood remains in the system from extraction to reinsertion.

In a first aspect, the present invention relates to a system for cell engineering target cells in a mixture comprising the target cells and non-target cells, comprising: i) a cell monitoring component for tracking and classifying individual cells in the mixture, wherein classifying a cell comprises determining whether the cell is a target cell; ii) an engineering component for selectively modifying individual cells at one or more engineering sites, wherein the target cells and the non-target cells are randomly distributed in the engineering component; and iii) a control component for controlling the engineering component so as to selectively modify only target cells tracked to be at one of the engineering sites.

In embodiments, the mixture may be a biological mixture, preferably a bodily fluid or a fraction thereof. The bodily fluid may for instance be blood or lymph. Although its non-therapeutic uses can likewise not be understated, the invention may in particular proof useful in cell therapy performed directly on bodily fluids or fractions thereof. For example, it provides a very effective way of performing procedures such as CAR-T immunotherapy.

In embodiments, the target cells may be immune cells or stem cells, preferably immune cells. In preferred embodiments, the immune cells may be peripheral blood mononuclear cells, more preferably lymphocytes, most preferably T cells or NK cells. Engineering these cell types advantageously has important therapeutic-but also non-therapeutic (e.g. research)-applications.

In embodiments, the cell monitoring component may comprise an imaging unit, preferably a computational microscopy module, most preferably a lens-free imaging unit. Compared to imaging using lenses, lens-free imaging typically allows for a considerably larger field of view (FoV; e.g. 15 mm² or more), thereby enabling longer (i.e. more spatially extended) tracking and monitoring. This longer tracking and monitoring can provide higher accuracy for cell identification, as well as for the selection of when to control the engineering component to selectively modify the target cells at one of the engineering sites. (Computational microscopy) lens-free imaging thus enables high-throughput (supporting relatively high flow rate) cell monitoring simultaneously over a wide area, thereby advantageously allowing efficient classification and tracking of the individual cells over the full cell monitoring zone using a single imaging unit (as opposed to e.g. needing to stitch together information/images from multiple imaging units). Alternatively, a plurality of the imaging units can nevertheless be used together-e.g. including stitching and integrating together the image of each-such that the imaging zone is increased, far beyond traditional imaging. This can further increase the yield of the system. Moreover, it enables label-free cell monitoring, thereby advantageously resolving the problems associated with cell labelling (cf. Background).

In embodiments, the imaging unit may comprise an illumination source and an image sensor. In embodiments, the illumination source (or `light source') may be configured for illuminating objects (e.g. cells) in the cell monitoring component (e.g. in the cell monitoring zone). For example, the light source may emit coherent light. Depending on the application, the emitted light may be monochromatic or polychromatic (as e.g. described in EP2879004A1 and/or LI, Yuqian, et al. Accurate label-free 3-part leukocyte recognition with single cell lens-free imaging flow cytometry. Computers in biology and medicine, 2018, 96: 147-156.; which are incorporated herein by reference). An interference pattern may thereby advantageously be formed by interference between light being scattered by the illuminated objects and non-scattered light from the illumination source. In embodiments, the image sensor (e.g. a digital image sensor) may be configured for capturing said interference pattern; e.g. as a digital hologram. In embodiments using computational imaging, the system may comprise a processor configured for receiving the captured interference pattern and processing it into an image. In embodiments, the system may comprise a processor configured for receiving one or more captured interference patterns a such or one or more of said images, and deriving from therefrom information (e.g. shape, size, transparency, scattering, speed, direction and/or path) the objects (e.g. cells) illuminated by illumination source. In embodiments, the aforementioned processors may be a single processor or may separate processors. In embodiments, the single or multiple processors may be (independently) comprised in the image sensor as such, the imaging unit, the control component or another external processor unit. Such an imaging unit does typically not require a lens, advantageously making it more compact (lenses and related optics make a microscope bulky), robust (lens alignments are not needed) and affordable. Moreover, it may-compared to traditional microscopes-typically advantageously have a comparable resolution but a much larger field of view (thus a much bigger imaged area). Notwithstanding, the computational imaging may in some embodiments nevertheless comprise a lens.

Notwithstanding, other types of cell monitoring-label-free or otherwise-are also within the scope of the present invention. For example, the cell monitoring component may comprise a more traditional image sensor, comprising a lens. In alternative or complementary embodiments, the cell monitoring component may comprise a plurality of image sensors. Multiple image sensors can allow imaging a larger area, even where the individual image sensors have relatively low field of view; by contrast keep track of individual cells can be more challenging as they move form one imaging window to the next. When using multiple image sensors, each image sensor may have its own dedicated illumination source, or two or more image sensors may share an illumination source.

In embodiments, the cell monitoring component may comprise a fluidic channel. In embodiments, the mixture may flow through the cell monitoring component via the fluidic channel. In embodiments, the fluidic channel may be a microfluidic channel. In embodiments, the system may be a microfluidic system (e.g. a microfluidic device). The use of compact (micro)fluidic channels and system/device architectures keeps the surface area with the which the mixture may interact low, thereby advantageously keeping the risk of contamination of the mixture to a minimum.

In embodiments, the cell monitoring component may comprise a cell movement modifying surface. In embodiments, the fluidic channel may comprise the cell movement modifying surface. The cell movement modifying surface advantageously allows (label-free) cell classification based on cell-surface interactions.

Cells may typically comprise cell membrane molecules which can act as cell identification molecules, such as antigens. Certain molecules, e.g. antibodies, may bind specifically to such cell membrane molecules. For example, antigen CD4 (cluster of differentiation 4) is a glycoprotein which binds to anti-CD4 antibodies. Accordingly, cells that bind to anti-CD4 antibodies may form one cell category. Such cells may be detected based on their modified movements (e.g. modified speed, direction, and/or path) when they travel through a zone with a cell movement modifying (CMM) surface coated by anti-CD4 antibodies. More specifically, the cells with antigen CD4 may undergo several bind-and-release events with anti-CD4 antibodies as they travel trough said zone. Analogously, cells that bind to anti-CD8 (cluster of differentiation 8) antibodies may form another cell category. Such cells may be detected based on their modified movements when they travel through a zone with a cell movement modifying surface coated by anti-CD8 antibodies. Binding and releasing the cell may temporarily halt the cell along its travel. This yields an altered motion in the form of a change in speed, direction and/or path, which is detectable and allows the cell to be classified.

In embodiments, the cell movement modifying surface may have an affinity for a particular type of cell over another type of cell, e.g. for a particular cell identification molecule over another cell identification molecule. For example, the cell movement modifying surface may comprise-or may be coated with-antibodies, aptamers, lectins and/or any other molecule that specifically binds to a cell. In embodiments, the cell modifying surface may bind the cell for a finite amount of time. In embodiments, the cell movement modifying surface may further be as described in EP23219766.5, which is incorporated herein by reference.

In embodiments, the cell monitoring component (e.g. the imaging unit) may be configured to detect an altered movement of a cell in the cell monitoring component (e.g. the cell monitoring zone); such as one or more bind-and-release events, a halt of motion, a change of speed, a change of direction, or a change of path. In some embodiments, the cell monitoring component (e.g. the imaging unit) may be configured to detect a threshold number (e.g. at least 2, preferably at least 5, more preferably at least 10) of the aforementioned altered movements. Regardless, the detected altered movement then in turn allows to classify the cell.

In embodiments, the fluidic channel may comprise one or more structures having the cell movement modifying surface. Said structures may generally have any shape; notwithstanding, the structures may in preferred embodiments be an array of pillars. Such structures can advantageously provide a high surface area for the cell-surface interactions. Notwithstanding, the use of such structures is not required and the cell movement modifying surface may also be provided on a (flat) surface of the fluidic channel.

In embodiments, the fluidic channel may comprise multiple subzones having distinct cell movement modifying surfaces. Using multiple distinct movement modifying surfaces advantageously allows additional degrees of selection, thereby raising the accuracy of the cell classification, and thereby the accuracy of the cell modification. For example, while the interactions of two similar cell types (e.g. having comparable physical and (bio)chemical properties) with any one movement modifying surface may be too similar to confidently tell apart, their differential interactions with a plurality of different movement modifying surfaces may typically provide a clearer picture and nevertheless allow them to be told apart. Additionally, it can allow for a plurality of types of cells (e.g. in a complex mixture) to be distinguished.

In embodiments, determining whether the cell is a target cell may be based on a determination of morphological parameters (e.g. shape and/or size), optical parameters (e.g. transparencyand/or scattering), and/or kinetic parameters (e.g. speed direction, and/or path) of the cell. Such properties advantageously allow to distinguish between different types (e.g. categories) of cells.

In embodiments, the engineering sites may be arranged with respect to the flow of the mixture such that every cell needs to pass at least one engineering site prior to exiting the engineering module. This advantageously ensures that the cell modification can be applied to every target cell. In other embodiments, not every cell passes at least one engineering site. For some applications, not all target cells need to be modified; e.g. the procedure may be effective (although possibly less so) if only a certain percentage (less than 100%) of the target cells are actually modified.

Tracking an individual cell may typically involve monitoring the position of said cell over time. In preferred embodiments, tracking of individual cells in the mixture (e.g. during and/or after classifying them) may be performed (at least) up to the engineering sites in the engineering component. By keeping track of classified cells up to the engineering component advantageously helps to ensure that only cells that are identified to be the target cell are modified in the engineering component. In other embodiments, tracking of individual cells in the mixture (e.g. during and/or after classifying them) may be performed up to a point prior to the engineering sites. This is typically possible if the movement of the tracked cells can be predicted from that point onwards. It can improve the efficiency of the system (e.g. by taking up less resources overall per tracked cell), but can be a source of errors if the actual cell movement for any reason deviates (too much) from the prediction. In some embodiments, only the target cells may continue to be tracked up to the engineering sites, while in other embodiments all cells may continue to be tracked up to the engineering sites. While tracking only the target cells may typically be sufficient to allow selectively modifying only the target cells, tracking also the non-target cells advantageously allows to additionally check that no non-target cells are at risk of being modified (e.g. because they are too close to a target cell).

Where tracking continues at least up to the engineering sites, the engineering component is typically at least partially (e.g. completely) contained within the cell monitoring component. More specifically, the cell monitoring component may comprise a cell monitoring zone (cf. supra and infra) and the engineering component may be at least partially (e.g. completely) in the cell monitoring zone. Moreover, the mixture may flow through the cell monitoring component (zone) and into the engineering component, and the cell classification may be performed in the cell monitoring component (zone) upstream compared to the engineering component. Where tracking is performed up to a point prior to the engineering sites, the engineering component is typically coupled (e.g. connected) to the cell monitoring component. Accordingly, the mixture may nevertheless still flow through the cell monitoring component (zone) and-optionally via a further zone/component-into the engineering component, and the cell classification may be performed in the cell monitoring component (zone) upstream compared to the engineering component.

In embodiments, selectively modify individual cells may comprise selectively inserting and/or modifying a nucleic acid in the individual cells. The modification of the cells may thus advantageously comprise a genetic transformation. In embodiments, selectively modifying the individual cells may comprise selectively transfecting the individual cells. Transfection advantageously enables efficient delivery of nucleic acids into the cells. In embodiments, transfecting may comprise electroporating, photoporating, cell squeezing, magnetofecting, chemical transfecting, or biochemical transfecting. This versatile selection of techniques is advantageously effective at opening (porating) the cell for modification (e.g. for genetic transformation).

In embodiments, a composition of the mixture may be maintained between classifying the individual cells and selectively modifying the individual cells (e.g. not including the selective modifying, as at least some reagents are typically added to the mixture at that point). In embodiments, nothing may be removed from the mixture between classifying the individual cells and selectively modifying the individual cells. Notwithstanding, in the latter embodiments, something may be added to the mixture (e.g. reagent, solvent, etc.). The selective modification is thus advantageously not based on cell sorting (e.g. separating the target cells from the non-target cells), but on individually selecting for modification only those cells classified as target cells and tracked to be at one of the engineering sites.

In embodiments, any feature of any embodiment of the first aspect may independently be as correspondingly described for any embodiment of any of the other aspects.

While the invention has hereto been described from the facet of a general system, the inventive elements may also advantageously be formulated as a module for integration into a larger system. Accordingly, in a second aspect, the present invention relates to a cell engineering module for cell engineering target cells in a mixture comprising the target cells and non-target cells, comprising: i) a cell monitoring zone for allowing tracking and classifying individual cells in the mixture, wherein classifying a cell comprises determining whether the cell is a target cell; and ii) an engineering component in the cell monitoring zone for selectively modifying individual cells at one or more engineering sites, wherein the target cells and the non-target cells are randomly distributed in the engineering component; wherein the engineering component is adapted for-in operation-being controlled so as to selectively modify only target cells tracked to be at one of the engineering sites.

In embodiments, the cell monitoring zone may be present within a fluidic channel. In embodiments, the mixture may flow through the cell monitoring zone via the fluidic channel. In embodiments, the fluidic channel may be a microfluidic channel. In embodiments, the cell engineering module may be a microfluidic module. The use of compact (micro)fluidic channels and system/module architectures keeps the surface area with the which the mixture may interact low, thereby advantageously keeping the risk of contamination of the mixture to a minimum.

In embodiments, the cell monitoring zone may comprise a cell movement modifying surface. In embodiments, the cell monitoring zone may comprise at least one transparent surface for imaging cells in the cell monitoring zone. The cell movement modifying surface may be, function and operate is described for the first aspect.

In embodiments, the cell monitoring zone may comprise at least one transparent surface- -preferably at least two opposing transparent surfaces-for imaging cells in the cell monitoring zone. Particularly when imaging unit is external to the cell engineering module, one or more transparent (e.g. substantially transparent to the wavelengths used for the cell imaging by the imaging unit) surfaces nevertheless allow tracking and classification in the cell monitoring component using the imaging unit.

In embodiments, the engineering component may be adapted for interfacing with a control component. For example, the control component may be coupled-e.g. wired or wirelessly-to an input of the engineering component. Interfacing with a control component enables automated selective cell modification in the module. This allows control over the selective modification of the engineering component by the control component (based on the classification and tracking in the cell monitoring component).

In embodiments, any feature of any embodiment of the second aspect may independently be as correspondingly described for any embodiment of any of the other aspects.

In a third aspect, the present invention relates to a cartridge comprising the cell engineering module according to any embodiment of the second aspect.

A cartridge format provides a convenient platform for the cell engineering module, advantageously allowing to easily dispose after use of those parts of the system which came into contact with the mixture. Conversely, the parts which do not come into contact with the mixture (e.g. the imaging unit and control component) can be integrated in a system adapted for receiving the cartridge (cf. infra), so that they can be easily reused (e.g. do not need to be disposed with the cartridge), thereby reducing the operation and hardware costs of the system.

In embodiments, any feature of any embodiment of the third aspect may independently be as correspondingly described for any embodiment of any of the other aspects.

In a fourth aspect, the present invention relates to a system comprising a cell engineering module as defined in any embodiment of the second aspect, or adapted for receiving a cartridge as defined in any embodiment of the third aspect.

In embodiments, system being adapted for receiving the cartridge may comprise the system comprising an interface adapted for receiving (e.g. 'slotting in') said cartridge.

In embodiments, the system may further comprise a control component for controlling the engineering component so as to selectively modify only target cells tracked to be at one of the engineering sites.

In embodiments, the system may further comprise an imaging unit, preferably a computational microscopy module, most preferably a lens-free imaging unit.

In embodiments, the system may further comprise-before the cell engineering module-an apheresis module for separating blood into a fraction of interest comprising the target cells, and a residual fraction. The apheresis module allows separating the fraction comprising the target cells from fractions not comprising the target cells, thereby reducing the complexity of the mixture subjected to the selective cell engineering. In embodiments, the apheresis may comprise leukapheresis, such as lymphapheresis. There are advantageously well-suited to separating white blood cells-or more specifically lymphocytes-, which are of particular interest to therapeutic and non-therapeutic uses of the present invention.

In embodiments, the system may comprise (e.g. before the cell engineering module and, if present, before the apheresis module) an extraction module for drawing blood from a patient. In embodiments, the system may comprise (e.g. after the cell selection and engineering module) a reinsertion module for transferring the modified target cells back into the patient.

In embodiments, the system may comprise a vein-to-vein system. Such a vein-to-vein system may in particular further comprise: -before the cell engineering module and, if present, before the apheresis module-the extraction module for drawing blood from a patient; and-after the cell selection and engineering module-the reinsertion module for transferring the modified target cells back into the patient. In embodiments, the system may be for performing the cell engineering in a closed loop wherein the blood remains within the system from the extraction to the reinsertion. Maintaining a closed system reduces contamination risks and reduces the quality control steps that need to be performed. Moreover, a closed-loop vein-to-vein further minimizes the contamination risk and the quality control steps.

In embodiments, the system may be a system for non-therapeutical applications (herein further referred to as a `lab system'). Compared to a vein-to-vein system, the lab system typically has additional degrees of freedom in its design. For example, the lab system does not generally need to provide for reinsertion into a patient. Moreover, the mixture does not need to come from a patient and can be any useful biological mixture, including artificial mixtures (e.g. cells in a buffer). The target cells can generally be any cells that are to be modified.

In embodiments, the system may further comprise a cell sorting module (e.g. before and/or after the cell engineering module). In embodiments, the system may further comprise a cell purification module (e.g. after the cell engineering module). Cell sorting and purification modules advantageously allow the (modified) target cells to separated from other components in the mixture (e.g. from contaminants, non-target cells and/or non-modified target cells).

In embodiments, the system may further comprise a flow generation unit (e.g. a pump) for inducing a flow of the mixture through the system (e.g. through the cell monitoring zone/component and engineering component).

In embodiments, the system may be for cell therapy. Although its non-therapeutic uses can likewise not be understated, the invention is particular beneficial in cell therapy, where it can address several of the issues currently faced in this field (cf. background).

In embodiments, any feature of any embodiment of the fourth aspect may independently be as correspondingly described for any embodiment of any of the other aspects.

In a fifth aspect, the present invention relates to a method for cell engineering target cells in a mixture comprising the target cells and non-target cells, comprising: a) classifying individual target cells in the mixture, wherein classifying a cell comprises determining whether the cell is a target cell; b) tracking at least some of the target cells; and c) selectively modifying at least some of the tracked target cells, wherein the target cells and the non-target cells are randomly distributed.

In embodiments, step b-and optionally step a-may comprise imaging of the individual target cells, preferably computational imaging, most preferably lens-free imaging. (Computational microscopy) lens-free imaging enables high-throughput (supporting relatively high flow rate) cell monitoring simultaneously over a wide area (large field of view), thereby advantageously allowing efficient classification and tracking of the individual cells over the full cell monitoring zone using a single imaging unit (as opposed to e.g. having to stitch together information/images from multiple imaging units). Moreover, it enables label-free cell monitoring, thereby advantageously resolving the problems associated with cell labelling (cf. Background).

In embodiments, selectively modifying at least some of the tracked target cells may comprise selectively inserting and/or modifying a nucleic acid in the tracked target cells. The modification of the cells may thus advantageously comprise a genetic transformation.

In embodiments, a composition of the mixture may be maintained throughout steps a, b, and up to c. In step c a such, at least some reagents are typically added to the mixture. In embodiments, nothing may be removed from the mixture throughout steps a, b, and c. Notwithstanding, in the latter embodiments, something may be added to the mixture (e.g. reagent, solvent, etc.) during steps throughout steps a, b, and up to c. The selective modification is thus advantageously not based on cell sorting (e.g. separating the target cells from the non-target cells), but on individually selecting for modification only those cells classified as target cells and tracked to be at one of the engineering sites.

In embodiments, any feature of any embodiment of the fifth aspect may independently be as correspondingly described for any embodiment of any of the other aspects.

The invention will now be described by a detailed description of several embodiments of the invention. It is clear that other embodiments of the invention can be configured according to the knowledge of the person skilled in the art without departing from the true technical teaching of the invention, the invention being limited only by the terms of the appended claims.

### Example 1: System for cell engineering target cells in a mixture

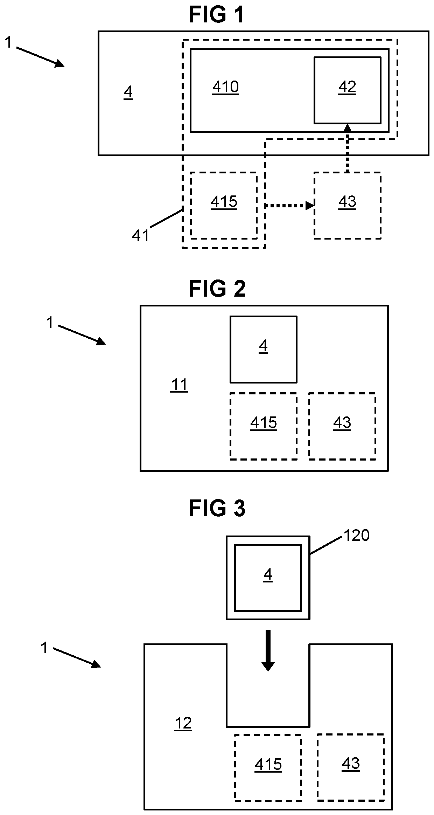

We now refer to FIG 1, schematically showing a system (1) in accordance with to the present invention. At the core of this system (1) is a cell engineering module (4), which comprises a cell monitoring zone (410) and-within the cell monitoring zone (410)-an engineering component (42). The cell monitoring zone (410) allows individual cells therewithin to be tracked and classified (including determining whether the cell is a target cell); while the engineering component (42) can selectively modify individual cells at one or more engineering sites (420). To this end, the engineering component (42) can be controlled so as to modify only selected individual cells.

In system (1) in operaton, tracking and classifying of the individual cells is typically performed using an imaging unit (415), such as a computational microscopy module. The cell monitoring zone (410) and the imaging unit (415) together form a cell monitoring component (41).

Moreover, an operational system (1) may further comprise a control component (43) for controlling the engineering component (42). For instance, the control component (43) may signal/instruct the engineering component (42) when to perform cell poration and/or when to release a (bio)chemical (e.g. a nucleic acid to be introduced in a cell), etc. The control component (43) therefor receives information from the imaging unit (415). Depending on the implementation, this could be processed information, such as tracking information on one or more target cells, or information that is to be further processed by the control component (43) (e.g. raw imaging data that is to be further processed to track and classify individual cells therein). Based on this received information, the control component (43) then ideally controls the engineering component (42) such that only target cells (i.e. determined to be such during the classification) which are tracked to be at one of the engineering sites are modified.

Depending on the implementation, the above modules and components may be organized with respect to the system (1) in different ways. For example, the system (1) may be a device (11) as schematically depicted in FIG 2, in which the cell engineering module (4)-and typically the imaging unit (415) and control component (43)-is (semi-)permanently incorporated (e.g. in principle removable-e.g. in case of repair-but not intended to be removed in normal use).

Alternatively, the cell engineering module (4) may be formulated as a cartridge (120), and the system (1) may be comprised of the cartridge (120) and a device (12) for receiving said cartridge (120); e.g. as schematically depicted in FIG 3. In this case, the imaging unit (415) and control component (43) may still be (semi-)permanently incorporated in the device (12), or they may likewise be formulated as parts which can be easily slotted in an out (not depicted in FIG 3). Regardless, where the cartridge (120) does not include the imaging unit (415), the cell monitoring zone (not visualized in FIG 3)-and the cartridge (120)-may generally be adapted for nevertheless allowing the imaging unit (415) to image the cell monitoring zone (410). To this end, the cell monitoring zone (410)-and the cartridge (120)-may for instance comprise at least one transparent surface (e.g. substantially transparent to the wavelengths used for the cell imaging by the imaging unit (415)).

### Example 2: Cell engineering module

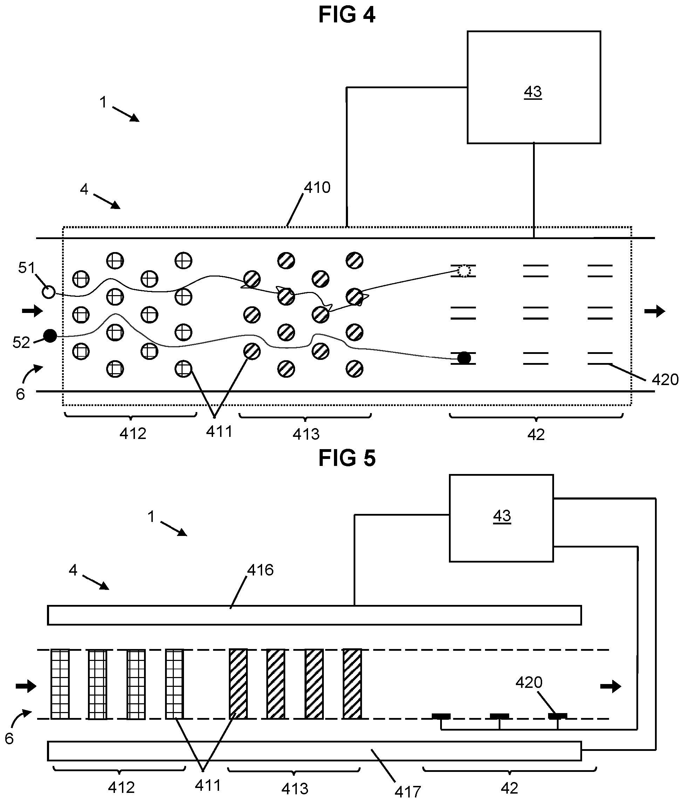

While a cell engineering module in accordance with the present invention could be realized in various ways, we now describe in more detail two exemplary approaches with reference to FIG 4-FIG 7.

### Example 2A: Selective cell poration

In a first exemplary approach, depicted in FIG 4 (top view) and FIG 5 (side view), the system (1)-more specifically the cell engineering module (4)-comprises a fluidic channel (6) through which the mixture comprising the target cells (51) and non-target cells (52) flows (left to right in FIG 4 and FIG 5). A cell monitoring zone (410) is present within (part of) this fluidic channel (6), wherein individual target cells (51) can be tracked and classified.

Typically this tracking and classifying is done using an imaging unit-such as a computational microscopy module, preferably a lens-free imaging unit-, which forms with the cell monitoring zone (410) a cell monitoring component (cf. supra). Depicted in FIG 5 is a lens-free imaging unit comprising an illumination source (416) and a digital image sensor (417). Moreover, the top wall and bottom wall of the fluidic channel (6) are optically transparent, such that light from the illumination source (416) can propagate into the cell monitoring zone (410) and to the digital image sensor (417). In operation, the illumination source (416) lights up the objects-e.g. cells-in the cell monitoring zone (410). Interaction of the light from the illumination source (416) with light that reflects off the objects thereby results in an interference pattern, which is captured by the digital image sensor (417) as a digital hologram. This captured interference pattern can be used as is (i.e. individual objects/cells can be tracked directly in the captured interference pattern), or can be computationally processed into a detailed image (e.g. including depth information) of the objects/cells. Either way, interpretation (e.g. tracking and/or classification) of the captured interference pattern and/or constructed image may be done by the imaging unit as such, or by the control component (43) (cf. infra).

As depicted in FIG 4 and FIG 5, the mixture flows in a front part (i.e. upstream) of the cell monitoring zone (410) along/across a cell movement modifying surface (411). More specifically, as depicted, the fluidic channel (6) comprises two subzones (412, 413), each outfitted with structures-namely an array of pillars-having different/distinct cell movement modifying surfaces (411). Depending on the nature of the cell, different cells may interact (e.g. temporarily bind) differently (e.g. showing a substantial difference in the number of bind-and-release events, the average duration of a bind-and-release, etc.) with the movement modifying surface(s) (411); thereby influencing their movement through the cell monitoring zone (410). By tracking the movement-e.g. the speed, direction and/or path and/or interaction times-of individual cells (e.g. using the imaging unit), information can be gained on the nature of said individual cells, which-optionally in combination with their shape and/or size (which can also be obtained using the imaging unit)-allows to classify the cells and determined whether the cell is a target cell (51). To that end, the one or more cell movement modifying surfaces (411) may typically be selected in function of the target cells (51) and non-target cells (52) which are expected to be present in the mixture, such that the influence on the movement of target cells (51) can be distinguished (e.g. in at least one of the subzones) from that of non-target cells (52). For instance, two individual cells may each follow a movement path-through the cell monitoring zone (410) to the engineering component (42)-as illustrated in FIG 4, in which both cells interact differently-especially in the second subzone-with the pillars having the movement modifying surface (411). Accordingly, by tracking them individually, it can be determined that one can be classified as a target cell (51) while the other is a non-target or non-classified cell (52).

Note that, while we have above described the classification of the individual cells based on cell movement and cell movement modifying surfaces (411), this is not always necessary. Indeed, depending on the target cells (51) and non-target cells (52) involved, their shape, size, transparency, scattering and/or speed as such (i.e. without using a cell movement modifying surface (411))-which can e.g. be obtained directly using the imaging unit-can already be sufficiently distinct to distinguish them.

Further downstream the cell monitoring zone (410), the mixture eventually flows through an engineering component (42). The engineering component (42) comprises engineering sites (420) at which individual cells can be selectively (e.g. under control by the control component (43), cf. infra) modified. Depending on the implementation, this may for instance involve (cf. supra) selectively opening the individual cell and/or triggering the release of one or more (bio)chemical for the modification. As depicted in FIG 4 and FIG 5, the engineering sites (420) are electrode pairs at which the selected cells can be electroporated. Reagents (e.g. (bio)chemicals) for the cell modification may be provided through one or more side channels (not visualized in FIG 4 or FIG 5; e.g. provided out of plane in FIG 4) fluidically coupled to the engineering component (42). Depending on the application at hand, these side channels can be one or more central side channels which provide the reagents to all (or at least a collection of) engineering sites (420), or a side channel per engineering site (420) for provide the reagents in a precise (e.g. selectively controlled) and local manner to each engineering site (420).

A control component (43) is moreover present which receives information from the cell monitoring component (41) (e.g. from the imaging unit) and-based on this information-controls the engineering component (42). The information received from the cell monitoring component (41) could be fully processed cell tracking and classification data, partially processed or unprocessed (e.g. the raw interference patterns or constructed images). For instance, the classification of individual cells may be done by the cell monitoring component (41) as such (e.g. by the imaging unit) or by the control component (43).

Accordingly, after classification (cf. supra), at least the cells determined to be target cells (51)-and optionally all cells-are tracked in the cell monitoring zone (410) until they arrive at one of the engineering sites (420). Once a target cell (51) is tracked to be at an engineering site (420), the engineering component (42) is activated by the control component (43) to selectively modify the target cell (51). For instance, in FIG 4, the electrode pair is triggered to electroporate the target cell (51) (indicated by the dotted line around the cell), thereby allowing the reagents for modifying the cell (e.g. by transfection) to enter the porated target cell (51). Conversely, non-target cells (52) do not trigger activation of the engineering component (42) and are left intact, so that reagents for modifying the cell cannot enter them and they thus do not undergo modification.

### Example 2B: Selective administration of reagent

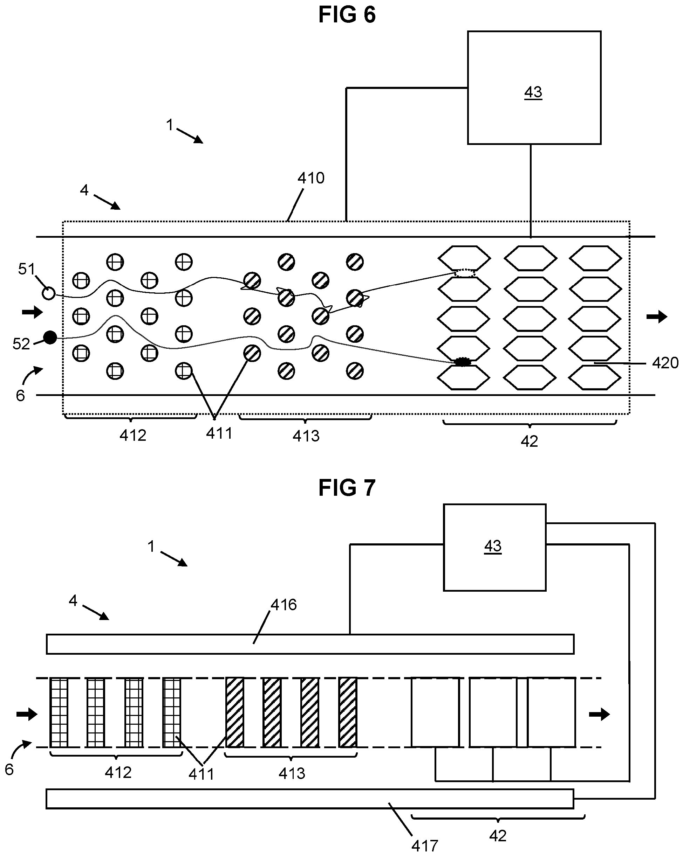

A second exemplary approach is depicted in FIG 6 (top view) and FIG 7 (side view) is substantially the same as the first approach, but-instead of electrode pairs for electroporation-the engineering sites (420) are narrow chokes at which all cells are porated through cell squeezing. Since in this approach all cells undergo poration, the selectivity of the modification is instead realized by selectively controlling-through the control module-the administration (e.g. release) of the reagents for modifying the cell such that it is available only locally at the engineering sites (420) where target cell (51) has been tracked to be. Accordingly, the one or more side channel (not visualized) for providing the reagents (e.g. (bio)chemicals) for the cell modification, may in this case typically comprise individual channel per engineering site.

### Example 2C: Selective administration of reagent

While not depicted separately, in a preferred implementation the selective cell poration of the first approach may be combined with the selective adminstration of reagent of the second approach. Accordingly, in this case the selectivity in ensured through both selectively opening the target cells and selectively locally adminstering necessary reagents. For instance, the control component (43) may activate the enigineering component (42) to trigger both the poration of a target cell (51) tracked to be at an engineering site (420) and the local release of one or more reagents at said eingineering site (420). This dual level of selectivity further ensures that only the selected cells undergo the desired modification.

### Example 3: Vein-to vein system

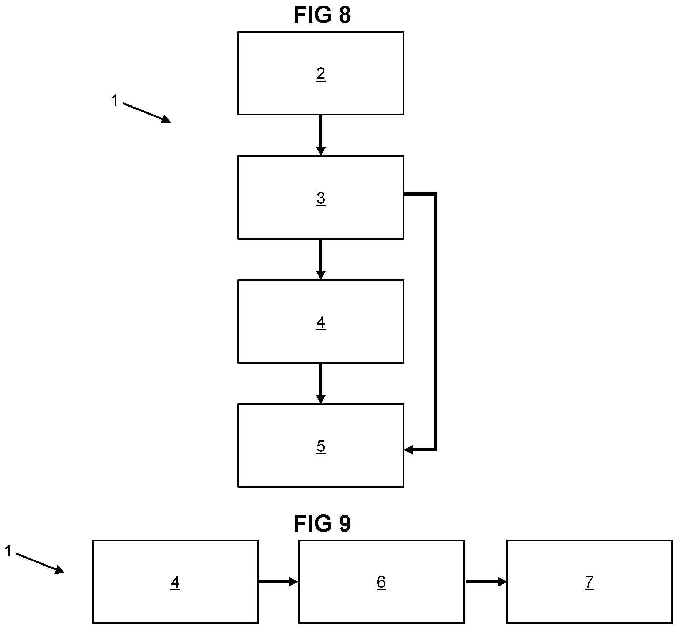

We now refer to FIG 8, which schematically depicts a vein-to-vein system in accordance with embodiments of the present invention. A vein-to-vein system is a particular example of a system (1) in accordance with the present invention (which is in general not limited to vein-to-vein), in which the mixture comprising the target cells and non-target cells is blood extracted (typically from a vein) from a patient and the modified target cells are later reintroduced into a patient (e.g. the same patient). Preferably, this is performed in a closed loop wherein the extracted blood remains in the system (1) from extraction to reinsertion. Moreover, the interaction of the extracted blood with any foreign (i.e. not native to the patient) products (e.g. reagents) and surfaces (e.g. in the system (1)) may preferably be kept to a minimum. These ensure that the risk of contamination of the extracted blood is kept low and thereby also relaxes the quality control that needs to be performed on the blood prior to reinsertion.

Such a vein-to-vein system (1) typically starts with an extraction module (2) for drawing blood from a patient.

From the extraction module (2), the extracted blood may go to an apheresis module (3) for separating it into a fraction of interest and a residual fraction. For example, where the target cells are white blood cells (e.g. T cells or NK cells), the apheresis module (3) may be a leukapheresis module which separates the fraction of white bloods cells from the residual blood.

The fraction of interest-which comprises the target cells, but nonetheless also non-target cells-then goes to the engineering module (4), where the target cells undergo cell engineering (cf. supra). After cell engineering, the fraction of interest goes to a reinsertion module (5).

The residual fraction is typically not provided to the engineering module (4), or at least is not subjected to the same cell modification in the engineering module (4) (e.g. it may enter the engineering module (4) but follow a trajectory separate from the fraction of interest). For instance, the residual fraction may bypass the cell engineering module (4) and be directly provided to the reinsertion module (5).

In the reinsertion module (5), the fraction of interest and the residual fraction are recombined and transferred back into the patient.

It will be clear that the above is but one implementation of a vein-to-vein system (1), and that the functionality may be added or deleted from the above modules and/or operations may be interchanged between modules. For example, in some embodiments the apheresis may not be needed and this module could be omitted. Moreover, the recombination of the fraction of interest and the residual fraction could be performed in a dedicated recombination module. A quality control module might also be added between the engineering module (4) and the reinsertion module (5), in which case the recombination could (but does not need to) take place in-or before-the quality control module. Alternatively, the quality control functionality could be integrated into the reinsertion module (5).

### Example 4: Lab system

We now refer to FIG 8, which schematically depicts one of many potential configurations for a lab system, e.g. to perform research or another (non-therapeutical) application. As depicted, the system (1) comprises an engineering module (4), a cell sorting module (6) and a cell purification module (7).

It is to be understood that although preferred embodiments, specific constructions, configurations and materials have been discussed herein in order to illustrate the present invention. It will be apparent to those skilled in the art that various changes or modifications in form and detail may be made without departing from the scope of the invention as defined in the appended claims.

## Claims

1. A cell engineering module (4) for cell engineering target cells (51) in a mixture (5) comprising the target cells (51) and non-target cells (52), comprising:
i) a cell monitoring zone (410) for allowing tracking and classifying individual cells (50) in the mixture (5), wherein classifying a cell (50) comprises determining whether the cell (50) is a target cell (51); and
ii) an engineering component (42) in the cell monitoring zone (410) for selectively modifying individual cells (50) at one or more engineering sites (420), wherein the target cells (51) and the non-target cells (52) are randomly distributed in the engineering component (42);
wherein the engineering component (42) is adapted for-in operation-being controlled so as to selectively modify only target cells (51) tracked to be at one of the engineering sites (420).

2. The cell engineering module (4) according to claim 1, wherein the cell monitoring zone (410) is present within a fluidic channel (6).

3. The cell engineering module (4) according to any of the previous claims, wherein the cell monitoring zone (410) comprises a cell movement modifying surface (411).

4. The cell engineering module (4) according to any of the previous claims, wherein the cell monitoring zone (410) comprises at least one transparent surface for imaging cells (50) in the cell monitoring zone (410).

5. The cell engineering module (4) according to any of the previous claims, wherein the engineering component (42) is adapted for interfacing with a control component (43).

6. The cell engineering module (4) according to any of the previous claims, wherein the target cells (51) are immune cells, preferably peripheral blood mononuclear cells, more preferably lymphocytes, most preferably T cells or NK cells.

7. A cartridge (120) comprising the cell engineering module (4) according to any of the previous claims.

8. A system (1)
- comprising (11) a cell engineering module (4) as defined in any of claims 1 to 6, or
- adapted for receiving (12) a cartridge (120) as defined in claim 7.

9. The system (1) according to claim 8, further comprising a control component (43) for controlling the engineering component (42) so as to selectively modify only target cells (51) tracked to be at one of the engineering sites (420).

10. The system (1) according to claim 8 or 9, further comprising an imaging unit (415), preferably a computational microscopy unit, most preferably a lens-free imaging unit.

11. The system (1) according to any of claims 8 to 10, further comprising-before the cell engineering module (4)-an apheresis module (3) (3) for separating blood into
- a fraction of interest comprising the target cells (51), and
- a residual fraction.

12. The system (1) according to claim 11, wherein the apheresis comprises leukapheresis.

13. The system (1) according to any of claims 8 to 12, being a vein-to-vein system further comprising:
- -before the cell engineering module (4) and, if present, before the apheresis module (3) (3)-an extraction module (2) (2) for drawing blood from a patient; and
- -after the cell engineering module (4)-a reinsertion module (5) (5) for transferring the modified target cells (51) back into the patient.

14. The system (1) according to claim 13, for performing the cell engineering in a closed loop wherein the blood remains within the system (1) from the extraction to the reinsertion.

15. The system (1) according to any of claims 8 to 14, for cell therapy.
